# EUROPÄISCHE PATENTSCHRIFT

(11) **EP 2 349 182 B1**
(45) Veröffentlichungstag und Bekanntmachung des Hinweises auf die Patenterteilung: **04.01.2017**
(21) Anmeldenummer: 09763918.1
(22) Anmeldetag: 24.11.2009
(51) Int. Cl.: A61K 8/04, A61K 8/06, A61K 8/39, A61K 8/44, A61K 8/46, A61K 8/894, A61Q 19/00, A61Q 17/04

(54) **SCHÄUMENDE EMULSIONEN**
FOAMING EMULSIONS
ÉMULSIONS MOUSSANTES

(30) Priorität: 27.11.2008 DE 102008059441
(43) Veröffentlichungstag der Anmeldung: 03.08.2011
(73) Patentinhaber: Henkel AG & Co. KGaA, 40589 Düsseldorf (DE)
(72) Erfinder: JANßEN, Frank, 41470 Neuss (DE); HENTREY, Stefanie, 20259 Hamburg (DE)
(86) Internationale Anmeldenummer: PCT/EP2009/065704
(87) Internationale Veröffentlichungsnummer: WO 2010/060894

(56) Entgegenhaltungen:
- EP-A2- 1 739 132
- WO-A1-03/088941

## Beschreibung

Die Erfindung betrifft kosmetische schäumende Pflegeemulsionen sowie deren Verwendung als Hautpflegemittel, sowie die Verwendung der Pflegeemulsion in einem manuell betätigbaren Schaumspender.

Schäumende Emulsionen sind im Stand der Technik im Prinzip bekannt, doch weisen diese oftmals ungünstige Eigenschaften wie schlechte Verteilbarkeit oder ein stumpfes Hautgefühl nach der Anwendung auf. Daher wurden in jüngster Zeit tensidhaltige Emulsionen entwickelt, die dank eines speziellen Pumpmechanismus mit Luft verschäumt werden und sich als Schaum leichter und gleichmäßiger auf der Haut verteilen lassen. Bei der Weiterentwicklung der verschäumbaren Emulsionen wurde jedoch festgestellt, dass der Schaum nicht fest genug war und schnell brach, wodurch auf der Haut nach der Anwendung ein wässriges und unangenehmes Gefühl hinterlassen wurde.

Um diese Nachteile zu beseitigen, wurde in der EP 1 496 852 vorgeschlagen, Polyolpoly-12-Hydroxystearate als Emulgatoren mit Tensiden, Ölkörpern, Lichtschutzfiltern und Wasser zu einer Emulsion in einem manuell betätigbaren Pumpspender zu verschäumen.

Auch diese Systeme konnten, insbesondere für den Zweck einer kosmetischen Hautpflegeemulsion, hinsichtlich ihrer Schaumqualität und Hautpflegeeigenschaften nicht zufriedenstellen, denn der entstehende Schaum war nicht dicht genug und wies eine rasch eintretende Drainage auf. Durch die Verwendung einer spezifischen Polymerkombination konnte die Schaumstabilität der schäumenden Emulsionen gemäß EP 1 496 852 zunächst verbessert werden. Anhand von Mikroskopaufnahmen war erkennbar, dass es sich um W/O/W-Emulsionen handelte, in der alle drei verschiedenen W/O/W-Micellentypen vorlagen, wobei W/O/W-Micellentyp C das System dominierte. Durch das Vorliegen der unterschiedlichen micellaren Strukturen resultierte aber eine extrem breite Partikelgrößenverteilung, wodurch der Schaum nach wie vor nicht dicht genug war und nach der Applikation zu einem stark klebrigen und stumpfen Hautgefühlt führte.

Aufgabe der vorliegenden Erfindung war es daher, eine kosmetische Pflegeemulsion herzustellen, die mittels eines manuell betätigbaren Schaumspenders aufschäumbar ist und einen sehr dichten, stabilen aber leichten Schaum bildet, der sich bequem auf der Haut verteilen lässt. Eine weitere Anforderung an die kosmetische Pflegeemulsion war es, bei der Verteilung des Schaums und nach der Absorption ein feuchtigkeitsspendendes, pflegendes Hautgefühl zu hinterlassen.

Überraschend wurde nun gefunden, dass sich diese Eigenschaften erreichen lassen, wenn man zwei spezielle Emulgatoren gleichen HLB-Werts sowie zwei spezielle Tenside zu einer Emulsion, die in einem manuell betätigbaren Schaumspender konfektioniert wird, miteinander kombiniert. Außergewöhnlich an der vorliegenden Erfindung ist, dass einer der Emulgatoren zwingend ein Silikonemulgator ist. Silikone werden üblicherweise chemischen Systemen hinzugefügt, um das Schäumen zu verhindern, daher war es nicht erwartet, den erfindungsgemäßen Effekt mit einem Silikonemulgator zu erzielen.

Gegenstand der Erfindung sind daher kosmetische Pflegeemulsionen gemäß Anspruch 1. Erfindungsgemäße Pflegeemulsionen entwickeln beim Aufschäumen einen besonders cremigen, dichten Schaum, der eine stark retardierte Drainage aufweist. Der Schaum hat eine leichte Textur und lässt sich sehr gut auf der Haut verteilen. Durch die Cremigkeit des Schaums werden die vorgenannten Nachteile wie Stumpfheit und Klebrigkeit nach der Absorption des Schaums beseitigt. Stattdessen spürt der Verbraucher bereits während der Verteilung die verbesserte Pflege, die sich nach der Absorption des Schaums in einem mit Feuchtigkeit gesättigten, glatten Hautgefühl ausdrückt.

Unter dem Begriff "Emulsion" wird erfindungsgemäß auf der einen Seite eine homogene Emulsion verstanden, in der die Wasser- und die Ölphase über einen sehr langen Zeitraum stabil ineinander emulgiert vorliegen. Gleichzeitig ist es erfindungsgemäß aber auch möglich, dass die "Emulsion" erst unmittelbar vor dem Aufschäumen (das bedeutet unmittelbar vor der Betätigung des Schaumspenders) hergestellt wird, indem die Öl- und die Wasserphase, die getrennt voneinander vorliegen, durch kräftiges Schütteln kurzzeitig ineinander emulgiert werden. Durch beide Ausführungsformen werden die erfindungsgemäßen Ziele erreicht.

Ein zweiter Gegenstand der Erfindung ist Verwendung einer Mischung aus einer Emulgatorkombination (A), enthaltend zwei W/O-Emulgatoren gleichen HLB-Werts, die ausgewählt sind aus:
(i) mindestens einem Poly(12-hydroxystearinsäure)polyglycerinester und
(ii) mindestens einem Silikonemulgator,
   einer Tensidkombination (B), enthaltend
(iii) mindestens ein anionisches Tensid und
(iv) mindestens ein amphoteres und/oder zwitterionisches Tensid, und
   1-25 Gew.-% mindestens einer Öl- oder Fettkomponente, bezogen auf das Gesamtgewicht der Pflegeemulsion,
zur Herstellung kosmetischer Pflegeemulsionen, die unter Verwendung eines manuellen Pumpspenders aufschäumbar sind und eine leichte Textur sowie eine bessere Verteilbarkeit aufweisen.

Ein dritter Gegenstand der Erfindung ist die Verwendung der erfindungsgemäßen Pflegeemulsion zur Verbesserung des Hautgefühls.

Ein vierter Gegenstand der Erfindung ist die Verwendung der erfindungsgemäßen Pflegeemulsion in einem Schaumspender mit Pumpmechanismus zur Verschäumung mit Luft.

Unter der Bezeichnung "den gleichen HLB-Wert" wird erfindungsgemäß verstanden, dass sich die HLB-Werte der Emulgatoren um weniger als 1, bevorzugt weniger als 0,8 und insbesondere weniger als 0,5 unterscheiden.

Die Emulgatorauswahl erfolgte sehr behutsam, denn es galt die vorgenannten Probleme zu beseitigen und bekannte Rezepturen zu verbessern. Die Emulgatoren sollten optimalerweise auch zu den pflegenden Eigenschaften der Pflegeemulsionen beitragen, ohne dass negative Wechselwirkungen mit anderen Bestandteilen oder Hautunverträglichkeiten auftraten.

Es wurde festgestellt, dass sich für diesen Zweck Emulgatoren eignen, die einen HLB-Wert im Bereich von 2 bis 8, bevorzugt von 3 bis 7 und insbesondere im Bereich von 4 bis 6 aufweisen. Emulgatoren mit einem HLB-Wert von 4,5; 4,6; 4,7; 4,8; 4,9; 5,0; 5,1; 5,2; 5,3; 5,4 oder 5,5 sind dabei besonders bevorzugt. Außerordentlich bevorzugt sind die Emulgatoren mit einem HLB-Wert von 4,8; 4,9; 5,0; 5,1 oder 5,2.

Auch das Verhältnis der Emulgatoren in der Pflegeemulsion spielt eine große Rolle zur Entfaltung aller positiven Eigenschaften der Pflegeemulsion. So wurden die besten Ergebnisse erzielt, wenn die Emulgatoren (i) und (ii) in einem Gewichtsverhältnis von 5 : 1 bis 1 : 4, bevorzugt von 3 : 1 bis 1 : 2 und insbesondere von 2 : 1 bis 1 : 1 in den Pflegeemulsionen eingesetzt wurden.

Die Emulgatoren (i) sind im Prinzip bekannte Stoffe, die beispielsweise unter den Bezeichnungen "Dehymuls^{®} PGPH", "Dehymuls^{®} SBL" oder "Eumulgin^{®} VL 75" von der Firma Cognis im Handel erhältlich sind.

Die Polyolkomponente dieser Emulgatoren weist mindestens zwei, bevorzugt 3 bis 12 und insbesondere 3 bis 8 Hydroxylgruppen sowie 2 bis 12 Kohlenstoffatome auf. Typische Beispiele sind:
- Glycerin und Polyglycerin,
- Alkylenglycole, wie beispielsweise Ethylenglycole, Diethylenglycol, Propylenglycol,
- Methyloylverbindungen, wie Trimethylolethan, Trimethylolpropan, Trimethylolbutan, Pentaerythrit und Dipentaerythrit,
- Alkylololigoglucoside mit 1 bis 22, bevorzugt 1 bis 16, mehr bevorzugt 1 bis 8 und insbesondere 1 bis 4 Kohlenstoffatomen im Alkylrest, wie Methyl- und Butylglucosid,
- Zuckeralkohole mit 5 bis 12 C-Atomen, wie beispielsweise Glucose oder Saccharose,
- Aminozucker, wie beispielsweise Glucamin.

Besonders bevorzugt aufgrund ihrer ausgezeichneten anwendungstechnischen Eigenschaften sind Umsetzungsprodukte auf der Basis von Polyglycerin und insbesondere die Umsetzungsprodukte von Poly-12-hydroxystearinsäure mit Polyglycerinen der folgenden Homologenverteilung:

| | |
|---|---|
| Glycerin: | 5 bis 35, insbesondere 15 bis 30 Gew.-%, |
| Diglycerin: | 15 bis 40, insbesondere 20 bis 32 Gew.-%, |
| Triglycerin: | 10 bis 35, insbesondere 15 bis 25 Gew.-%, |
| Tetraglycerin: | 5 bis 20, insbesondere 8 bis 15 Gew.-%, |
| Pentaglycerin: | 2 bis 10, insbesondere 3 bis 8 Gew.-%, |
| Oligoglycerin: | ad 100 Gew.-%. |

Erfindungsgemäß insbesondere bevorzugt ist ein Poly(12-hydroxysterinsäure)polyglycerinester, der von der Firma Cognis im Handel unter der Bezeichnung "Dehymuls^{®} PGPH" als W/O-Emulgator angeboten wird. Dehymuls® PGPH weist nach Herstellerangaben einen HLB-Wert von etwa 5 auf. Er wird in den erfindungsgemäßen Pflegeemulsionen - bezogen auf ihr Gesamtgewicht-in einer Menge von 0,05 bis 20 Gew.-%, bevorzugt 0,075 bis 15 Gew.-%, mehr bevorzugt von 0,1 bis 12 Gew.-% und insbesondere von 0,15 bis 10 Gew.-% eingesetzt.

Der zweite zwingende Emulgator in den erfindungsgemäßen Pflegeemulsionen ist ein Silikonemulgator, insbesondere ein Organopolysiloxan-Polyoxyalkylen-Emulgator, der-bezogen auf das Gesamtgewicht der Pflegeemulsionen - in einer Menge von 0,05 bis 15 Gew.-%, bevorzugt von 0,075 bis 12 Gew.-%, mehr bevorzugt von 0,1 bis 10 Gew.-% und insbesondere von 0,15 bis 7 Gew.-% eingesetzt wird.

Erfindungsgemäß geeignete Organopolysiloxan-Polyoxyalkylen-Emulgatoren entsprechen der allgemeinen Formel (I), 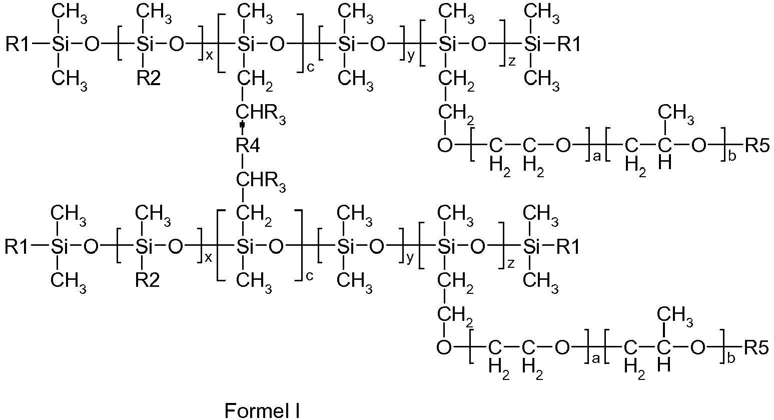 in der
die Reste R1 unabhängig voneinander aliphatische Reste mit 1 bis 25 Kohlenstoffatomen,
die Reste R2 unabhängig voneinander aliphatische Reste mit 2 bis 25 Kohlenstoffatomen,
die Reste R3 unabhängig voneinander Wasserstoff oder aliphatische Reste mit 1 bis 3 Kohlenstoffatomen,
der Rest R4 eine anorganische oder Organosiloxan-Gruppe ist, die keine hydrolysierbaren Bindungen enthält,
die Reste R5 unabhängig voneinander Endgruppen sind, die mit den Bestandteilen, die den Emulgator stabilisieren sollen, nicht nachteilig reagieren und die Bildung des Organopolysiloxan-Polyoxyalkylens nicht stören, stehen, und
x für einen Wert von 1 bis 100,
y für einen Wert von 0 bis 600,
z für einen Wert von 1 bis 100 steht, wobei x+y+z mindestens 30 ist,
a für einen Wert von 4 bis 40,
b für einen Wert von 0 bis 40 und
c für einen Wert von 0 bis 5 steht, wobei das Verhältnis von a : b 20 : 80 bis 100 : 0 ist.

Erfindungsgemäß besonders bevorzugt sind Organopolysiloxan-Polyoxyalkylen-Emulgatoren, in denen R1 für Methyl, R2 für C₈-C₁₈-Alkyl, R3 für Wasserstoff, R4 für -(CH₃)₂-Si-O-Si-(CH₃)₂-, R5 für Wasserstoff, x für 5 bis 60, y für 0 bis 150, z für 1 bis 15, a für 10 bis 30, b für 10 bis 30 und c für 0 oder 1 steht. Insbesondere bevorzugt sind solche Organopolysiloxan-Polyoxyalkylen-Emulgatoren, in denen R2 für C16-Alkyl, x für 5 bis 50, y für 25 bis 150, z für 1 bis 15 und c für 0 steht und das Verhältnis von a : b bei 40 : 60 bis 70 : 30 liegt.

Ein Beispiel für einen solchen insbesondere bevorzugten Silikonemulgator ist der unter der INCI-Bezeichnung "Cetyl PEG/PPG-10/1-Dimethicone" (vormals: Cetyl Dimethicone Copolyol) und dem Handelsnamen "Abil EM 90" von der Firma Goldschmidt vertriebene W/O-Emulgator. Abil® EM 90 weist nach Herstellerangaben einen HLB-Wert von etwa 5 auf.

Beide erfindungsgemäß bevorzugten Emulgatortypen sind dafür bekannt, dass man mit ihnen besonders stabile W/O/W-Emulsionen herstellen kann, weshalb erwartet wurde, dass sich durch die Kombination der Emulgatoren eine stabile W/O/W-Emulsion vom Typ B oder C ausbildet. Dies war aber nicht der Fall, wie aus Mikroskopaufnahmen deutlich erkennbar war. Die mikroskopische Betrachtung zeigte, dass es sich bei den erfindungsgemäßen Emulsionen um eine Kombination verschiedener Emulsionssysteme handelte. Man konnte sowohl W/O/W-Emulsionszellen des Typs A, als auch O/W-Emulsionszellen erkennen, in denen sich die Größe der Emulsionszellen parallel zum Emulsionstyp deutlich verändert hatte. Die Micellengröße der W/O/W-Emulsionszellen des Typs A war im Vergleich zu den Micellengrößen, die mit Dehymuls PGPH als alleinigem Emulgator ausgebildet wurden, wesentlich geringer.

Daraus resultierte - trotz der verschiedenen Emulsionstypen in den erfindungsgemäßen Pflegeemulsionen - eine sehr enge Partikelgrößenverteilung mit außerordentlich hoher Packungsdichte, was für die Bildung des cremigen, pflegenden Schaums essentiell ist.

In einer bevorzugten Ausführungsform umfasst die erfindungsgemäße Pflegeemulsion daher verschiedene Emulsionstypen, die ausgewählt sind aus W/O/W-Emulsionen des Typs A, B und C, insbesondere des Typs A, sowie O/W-Emulsionszellen.

Zur Erhöhung der Schaumbildung und -stabilisierung enthalten die erfindungsgemäßen Pflegeemulsionen zwingend Tenside anionischer sowie amphoterer und/oder zwitterionischer Natur. Auch bei den Tensiden musste eine behutsame Auswahl stattfinden, denn einerseits sollten die Tenside besonders mild sein, um eine hautschonende Behandlung ohne das Auftreten von Hautirritationen zu gewährleisten, und andererseits sollten die Tenside eine ausreichende Schaumbildung gewährleisten, um für die erfindungsgemäße Applikation in einem manuell betätigbaren Schaumspender geeignet zu sein.

Als erfindungsgemäß geeignete Tensidkombination zur Erfüllung der vorgenannten Erfordernisse haben sich milde anionische und milde amphotere/zwitterionische Tenside herausgestellt, die ausgewählt sind aus einem oder mehreren Tensiden der Gruppe (iii) der Acylaminosäuren, alpha-Olefinsulfonate, ethoxylierten Alkylschwefelsäurester, Sulfobernsteinsäureester, Ethercarbonsäuren und/oder den Salzen dieser Verbindungen, sowie aus einem oder mehreren Tensiden der Gruppe (iv) der Alkylbetaine, Sulfobetaine, Alkylamidoalkylbetaine, Aminopropionate, Aminoglycinate und/oder Imidazoliniumbetaine und/oder den Salzen dieser Verbindungen.

Unter den erfindungsgemäß geeigneten anionischen Tensiden (iii) sind Verbindungen zu verstehen, die unter den Natrium-, Kalium- und Ammonium- sowie der Mono-, Di- und Trialkanolammoniumsalzen mit 2 bis 4 C-Atomen in der Alkanolgruppe, der
- Ethercarbonsäuren der Formel R-O-(CH₂-CH_{2O})ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Acylglutamate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alpha-Olefinsulfonate mit 8 bis 24 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12,
ausgewählt sind.

Besonders geeignete anionische Tenside (iii) in den erfindungsgemäßen Pflegeemulsionen sind aufgrund ihrer ausgezeichneten Milde und Schäumbarkeit die Sulfobernsteinsäuremonoester-Salze von Fettalkoholen und Fettalkoholpolyglycolethern der allgemeinen Formel

R1-O-(CH₂-CH₂-O)ₙ-CO-CH₂-CH(SO₃⁻)-COO⁻ M₂⁺,

in der R1 eine Alkylgruppe mit 12 bis 18 Kohlenstoffatomen, n einen Mittelwert von 0-6 aufweist und M ein Alkalikation, bevorzugt Natrium, ist. Insbesondere geeignet sind Sulfobernsteinsäuremonoester-Salze der Formel

R1-O-(CH₂-CH₂-O)ₙ-CO-CH₂-CH(SO₃Na)-COONa,

in der R1 eine Alkylgruppe mit 12 bis 16 Kohlenstoffatomen und n einen Mittelwert von 1-6 aufweist. Solche Produkte mit n=3 sind unter der Handelsbezeichnung Texapon^{®} SB 30 von der Firma Cognis im Handel erhältlich.

Das oder die anionische(n) Tensid(e) wird (werden) in den erfindungsgemäßen Pflegeemulsionen - bezogen auf ihr Gesamtgewicht - bevorzugt in Mengen von 0,1 bis 20 Gew.-%, bevorzugt von 0,2 bis 15 Gew.-% und insbesondere in Mengen von 0,5 bis 9 Gew.-% eingesetzt.

Erfindungsgemäß geeignete zwitterionische Tenside sind die sogenannten Betaine wie die Alkylbetaine, N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat.

Ein erfindungsgemäß bevorzugtes zwitterionisches Tensid ist das unter der INCI-Bezeichnung Cocamidopropyl Betaine bekannte Fettsäureamid-Derivat.

Beispiele für erfindungsgemäß geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Erfindungsgemäß bevorzugte ampholytische Tenside sind das N-Kokosalkylaminopropionat, das Kokosacylaminoethylaminopropionat und das C₁₂ - C₁₈ - Acylsarcosin.

Besonders geeignete amphotere und/oder zwitterionische Tenside (iv) in den erfindungsgemäßen Pflegeemulsionen sind aufgrund ihrer ausgezeichneten Milde und Schäumbarkeit Ampho- und Betaintenside, die eine Alkyl- bzw. Acylgruppe mit 8 bis 10 Kohlenstoffatomen aufweisen, insbesondere Betaintenside der Formel

R2CONH(CH₂)ₘ-N⁺(CH₃)₂-COO⁻,

in der R2CO eine von Capryl- und Caprinsäure abgeleitete Gruppe und m=3 ist. Ein solches Tensid ist als besonders haut- und schleimhautfreundliches sowie reizarmes Betaintensid bekannt, und unter der Bezeichnung Tego Betain 810 von der Firma Goldschmidt im Handel erhältlich.

Das oder die amphotere(n) und/oder zwitterionische(n) Tensid(e) wird (werden) in den erfindungsgemäßen Pflegeemulsionen - bezogen auf ihr Gesamtgewicht - bevorzugt in Mengen von 0,1 bis 20 Gew.-%, bevorzugt von 0,2 bis 15 Gew.-% und insbesondere in Mengen von 0,5 bis 9 Gew.-% eingesetzt.

In einer weiteren bevorzugten Ausführungsform der Erfindung werden den Pflegeemulsionen zusätzlich zu den vorgenannten zwingenden Komponenten weiterhin Stoffe zur Erhöhung der Emulsions- und Schaumstabilität zugesetzt. Es wurde gefunden, dass sich eine besonders gute Schaumstabilisierung erzielen lässt, wenn eine Kombination verschiedener Polymere verwendet wurde. Als insbesondere geeignet für die erfindungsgemäßen Pflegeemulsionen hat sich die Kombination aus mindestens zwei Polymeren erwiesen, von denen eines natürlichen und das andere synthetischen Ursprungs ist.

Erfindungsgemäß geeignete Polymere natürlichen Ursprungs basieren auf Xanthan Gum, Guar Gum, Alginaten und/oder Carrageenaten. Als besonders bevorzugt haben sich Polymere herausgestellt, die sich aus Alginaten herstellen lassen, wobei insbesondere veresterte Alginate erfindungsgemäß geeignet sind. Neben der schaum- und emulsionsstabilisierenden Wirkung haben diese Verbindungen den Vorteil, dass sie dazu beitragen können, den Wasserverlust in der Haut reduzieren, wodurch sie die feuchtigkeitsspendende Wirkung der erfindungsgemäßen Pflegeemulsionen zusätzlich unterstützen.

Besonders geeignete Alginatester werden aus Alginaten hergestellt, die mit einem oder mehreren C₁-C₆-Alkoholen, welche eine oder mehrere Hydroxygruppen tragen können, verestert werden. Insbesondere geeignet sind Propylenglycolalginate, die durch die Umsetzung eines Alginats mit Propylenoxid bei hohen Temperaturen entstehen. Solche veresterten Alginate sind beispielsweise von der Firma FMC Biopolymers unter der Bezeichnung "Protanal Ester" im Handel erhältlich. Protanal Ester^{®} CF ist für die erfindungsgemäße Pflegeemulsion besonders bevorzugt, denn er weist in wässrigen, alkoholischen oder wässrig alkoholischen Lösungen eine niedrige Viskosität auf, was sich auf die leichte Verschäumbarkeit der Pflegeemulsionen positiv auswirkt.

Die Polymere natürlichen Ursprungs werden in den erfindungsgemäßen Pflegeemulsionen - bezogen auf deren Gesamtgewicht - bevorzugt in Mengen von 0,01 bis 10 Gew.-%, besonders bevorzugt von 0,05 bis 5 Gew.-% und außerordentlich bevorzugt in Mengen von 0,1 bis 2 Gew.-% eingesetzt.

Geeignete synthetische Polymere sind ausgewählt aus anionischen Homo- und Copolymeren aus unvernetzten und vernetzten Polyacrylsäuren, die teilweise oder vollständig neutralisiert sind.

Besonders bevorzugte anionische Homo- und Copolymere sind hierbei vernetzte Polyacrylsäuren, die teilweise oder vollständig neutralisiert sind. Solche Polymere sind zum Beispiel die Handelsprodukte der Carbopol^{®}-Serie, wie Carbopol^{®} 934, 940, 941, Carbopol^{®} Ultrez 10, Carbopol^{®} ETD 2001 und 2050 sowie die Produkte Tego Carbomer 140 und 141 und Synthalen^{®} L.Die Polyacrylsäuren werden in den erfindungsgemäßen Pflegeemulsionen - bezogen auf deren Gesamtgewicht - bevorzugt in einer Menge von 0,01 bis 4 Gew.-%, besonders bevorzugt von 0,02 bis 2 Gew.-% und außerordentlich bevorzugt in einer Menge von 0,03 bis 1 Gew.-% eingesetzt.

Weitere erfindungsgemäß bevorzugte synthetische Polymere sind Polyaryloyldimethyltaurate, wie beispielsweise die vernetztenAcryloyldimethyltaurat-Acrylamid-Copolymere mit dem Handelsnamen Sepigel^{®} 305 oder Simulgel^{®} 600 der Firma Seppic, die Homopolymere der 2-Acrylamido-2-methylpropansulfonsäure (Acyloyldimethyltaurin), die gegebenenfalls vernetzt und/oder neutralisiert sein können, wie beispielsweise Poly(2-acrylamido-2-methylpropansulfonsäure), die von der Firma Hoechst unter dem Handelsnamen Hostacerin^{®} AMPS oder von der Firma Seppic unter dem Handelsnamen Simulgel^{®} 800 vertrieben wird, und Copolymere der 2-Acrylamido-2-methylpropansulfonsäure und Hydroxyethylacrylate, wie beispielsweise die unter den Handelnamen Sepinov^{®} EMT 10 oder Simulgel^{®} NS von der Firma Seppic vertriebenen Handelsprodukte.

Die Polyacryloyldimethyltaurate werden in den erfindungsgemäßen Pflegeemulsionen - bezogen auf deren Gesamtgewicht - bevorzugt in einer Menge von 0,01 bis 5 Gew.-%, besonders bevorzugt von 0,02 bis 2 Gew.-% und außerordentlich bevorzugt in einer Menge von 0,05 bis 1 Gew.-% eingesetzt.

Als besonders geeignet für die Herstellung der erfindungsgemäßen Pflegeemulsionen hat sich die Kombination einer Polyacrylsäure mit einem Polyacryloyldimethyltaurat erwiesen, wobei die beste Schaum- und Emulsionsstabilisierung durch die Kombination der beiden Polymere Tego Carbomer^{®} 140 und Sepinov^{®} EMT 10 erzielt werden konnte.

In einer besonders bevorzugten Ausführungsform der Erfindung eignet sich als Polymerkombination zur optimalen Schaum- und Emulsionsstabilisierung insbesondere die Kombination eines Alginatesters mit einer Polyacrylsäure und einem Polyacryloyldimethyltaurat, und insbesondere die Kombination der Handelsprodukte Protanal Ester^{®} CF, Tego Carbomer^{®} 140 und Sepinov^{®} EMT 10.

Die zur Herstellung der erfindungsgemäßen Pflegemulsionen ebenfalls zwingend erforderliche Ölkompomponente muss hautpflegende Eigenschaften aufweisen, mit den vorgenannten, zwingenden Komponenten leicht emulgierbar sein und die Emulsionsstabilität nicht negativ beeinflussen. Des Weiteren darf die Ölkomponente die Schaumentwicklung sowie die Sensorik des Schaums nicht beeinträchtigen. Unter diesen Voraussetzungen sind sie folgenden natürlichen und synthetischen Ölkomponenten und/oder Fettstoffe erfindungsgemäß geeignet:
- natürliche (pflanzliche) Öle: dabei handelt es sich üblicherweise um Triglyceride und Mischungen von Triglyceriden. Bevorzugte natürliche Öle im Sinne der Erfindung sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Kakaoabutter und Shea-Butter.
- mineralische Öle: als mineralische Öle kommen insbesondere Mineralöle, Paraffin- und Isoparaffinöle sowie synthetische Kohlenwasserstoffe zum Einsatz. Ein erfindungsgemäß einsetzbarer Kohlenwasserstoff ist beispielsweise das als Handelsprodukt erhältliche 1,3-Di-(2-ethylhexyl)-cyclohexan (Cetiol^{®} S).
- snthetische Öle: als synthetische Öle kommen auch Silikonverbindungen wie Cyclomethicone, Dimethicone und Amodimethicone in Betracht.

Als Ölkomponente kann weiterhin ein Dialkylether dienen.

Erfindungsgemäß einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyln-undecylether sowie Di-tert.-butylether, Di-iso-pentylether, Di-3-ethyldecylether, tert.-Butyl-n-octylether, iso-Pentyl-n-octylether und 2-Methylpentyl-n-octylether.

Erfindungsgemäß besonders bevorzugt ist der Di-n-octylether, der im Handel unter der Bezeichnung Cetiol^{®} OE erhältlich ist.

Unter erfindungsgemäß geeigneten Fettstoffen sind Fettsäuren, Fettalkohole sowie natürliche und synthetische Wachse zu verstehen, welche sowohl in fester Form als auch flüssig in wässriger Dispersion vorliegen können.

Als Fettsäuren können eingesetzt werden lineare und/oder verzweigte, gesättigte und/oder ungesättigte Fettsäuren mit 6-30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Als Fettalkohole können eingesetzt werden gesättigte, ein- oder mehrfach ungesättigte, verzweigte oder unverzweigte Fettalkohole mit C₆-C₃₀-, bevorzugt C₁₀-C₂₂- und ganz besonders bevorzugt C₁₂-C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnussöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanetteo, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden. Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Weitere Fettstoffe sind beispielsweise
- Esteröle. Unter Esterölen sind zu verstehen die Ester von C₆-C₃₀ - Fettsäuren mit C₂-C₃₀-Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, Glycerincarbonat oder Dicaprylylcarbonat (Cetiol^{®} CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls^{®} 90-018, Monomuls^{®} 90-L12, Cetiol^{®} HE oder Cutina^{®} MD, und
- Ester der Benzoesäure mit linearen und/oder verzweigten C₆-C₂₂-Alkoholen, wie beispielsweise das unter der Handelsbezeichnung Finsolv^{®} TN vertriebene C₁₂-C₁₅-Alkylbenzoat.

Die Cremigkeit und die Feuchtigkeit- und Glätte-spendenden Eigenschaften werden besonders positiv beeinflusst, wenn als Ölkomponenten Dialkylether, Dialkylcarbonate und Benzoesäureester eingesetzt werden. Erfindungsgemäß besonders geeignet war eine Kombination aus Dicaprylylether (z.B. Cetiol^{®} OE), Ethylhexyl Palmitate (z.B. Cegesoft^{®} C24) und C₁₂-C₁₅-Alkylbenzoat (z.B. Finsolv^{®} TN).

Die Gesamtmenge der vorgenannten Öl- und Fettkomponenten in den erfindungsgemäßen Pflegeemulsionen beträgt - bezogen auf das Gesamtgewicht der Pflegeemulsionen - 1 - 25 Gew.-%, bevorzugt 3 bis 20 Gew.-%, mehr bevorzugt 5 bis 15 Gew.-% und insbesondere 6 bis 10 Gew.-%.

Falls die erfindungsgemäßen Pflegeemulsionen neben den vorgenannten Öl- und Fettkomponenten weitere hydrophobe Wirkstoffe, wie insbesondere Lichtschutzfilter, enthalten soll, ist es besonders bevorzugt, dass die Gesamtmenge der emulgierten Ölphase, einschließlich der vorgenannten Öl- und Fettkomponenten und weiterer hydrophober Wirkstoffe in den erfindungsgemäßen Pflegeemulsionen - bezogen auf das Gesamtgewicht der Pflegeemulsionen - 1-25 Gew.-%, bevorzugt 3 bis 20 Gew.-%, mehr bevorzugt 5 bis 15 Gew.-% und insbesondere 6 bis 10 Gew.-% beträgt.

Besonders bevorzugte erfindungsgemäße Pflegeemulsionen sind dadurch gekennzeichnet, dass die vorgenannten Öl- und Fettkomponenten in einer Gesamtmenge von bevorzugt 3 bis 20 Gew.-%, besonders bevorzugt 5 bis 15 Gew.-% und insbesondere 6 bis 10 Gew.-%, und weitere hydrophobe Wirkstoffe, wie insbesondere Lichtschutzfilter, in einer Gesamtmenge von 0,5 - 22 Gew.-%, bevorzugt 1-15 Gew.-%, besonders bevorzugt 3-12 Gew.-%, und außerordentlich bevorzugt 5-10 Gew.-% enthalten sind, jeweils bezogen auf das Gesamtgewicht der Pflegeemulsion.

Die Einwaage der obligatorischen Emulgatoren (i) und (ii) wird nicht zur Ölphase gerechnet.

Die Menge der Wasserphase in den erfindungsgemäßen Emulsionen beträgt bevorzugt > 35 Gew.-%, mehr bevorzugt > 45 Gew.-%, besonders bevorzugt > 55 Gew.-% und insbesondere bevorzugt > 65 Gew.-% jeweils bezogen auf das Gesamtgewicht der Emulsion.

Neben den bereits genannten, zwingenden Komponenten und fakultativen Komponenten, die zu besonders bevorzugten Ausführungsformen der erfindungsgemäßen Pflegeemulsionen gehören, enthalten die Pflegeemulsionen zur weiteren Steigerung der Haut pflegenden und schützenden Wirkung mindestens einen oder mehrere Hilfs- und Wirkstoffe, die ausgewählt sind aus der Gruppe der UV-Lichtschutzfilter, Antioxidantien, Feuchthaltemittel, Hyaluronsäure sowie deren physiologisch verträglichen Salzen und Derivaten, Wirkstoffen zur Stimulation der Kollagensynthese, Wirkstoffen zur Erhöhung bzw. Verbesserung der Interaktion zwischen der extrazellulären Matrix und den Fibroblasten sowie Wirkstoffen zur Förderung der Elastinproduktion.

Weitere erfindungsgemäß bevorzugte Pflegeemulsionen sind dadurch gekennzeichnet, dass sie zusätzlich zu der zwingenden Wirkstoffkombination nach Anspruch 1 mindestens einen die Kollagensynthese stimulierenden Wirkstoff enthalten, der ausgewählt ist aus
- Retinoiden, insbesondere Retinol und C₂-C₂₂-Fettsäureestern des Retinols,
- Kombucha,
- Olivenblattextrakten (Olea Europaea (Olive) Leaf Extract),
- Oleanolsäure,
- Oleanol,
- Grüntee-Extrakten (Camellia Sinensis),
- Kreatin,
- sowie Mischungen der vorgenannten Substanzen.

Zu den Retinoiden werden im Sinne der vorliegenden Erfindung Retinol (Vitamin A₁), die C₂-C₂₂-Fettsäureester des Retinols (= Retinylester, insbesondere Retinylpalmitat und Retinylacetat), 3,4-Didehydroretinol (Vitamin A₂), Retinal und seine Isomere, a*ll*-trans-Retinsäure, 9-*cis*-Retinsäure und 13-*cis*-Retinsäure (Tretinoin), die Ester der Retinsäure sowie weitere verwandte Substanzen (synthetische Retinoide) gezählt, die für ihre vielfältige biologische Wirkung insbesondere auf Wachstum und Differenzierung bekannt sind, sowie weiterhin Substanzen, die eine spezifische biologische Wirkung durch die Bindung an die Retinoid-Rezeptoren RAR (retinoid acid receptor) und RXR (retinoid X receptor) aufweisen und deren Aktivierung verursachen. Synthetische Retinoide werden in drei Gruppen eingeteilt: nichtaromatisch (z. B. *Isotretinoin),* monoaromatisch (z.B. Acitretin) und polyaromatisch (sogenannte Arotinoide, z. B. Tazarotene, die eine spezifische Wirkung auf einzelne Retinoid-Rezeptoren haben). Erfindungsgemäß besonders bevorzugte Retinoide sind Retinol und die C₂-C₂₂-Fettsäureester des Retinols, insbesondere Retinylpalmitat und Retinylacetat.

Weitere erfindungsgemäß bevorzugte Wirkstoffe, die die Kollagensynthese stimulieren, sind ausgewählt aus Kombucha.

Bei Kombucha handelt es sich um ein aus dem ostasiatischen Raum stammendes, etwa 0,5 Vol.-% Alkohol enthaltendes, angenehm säuerliches, leicht süßes, moussierendes Gärungsgetränk auf Basis von Mono- oder Disaccharid-haltigem Tee (Schwarzer Tee, Grüner Tee, Kräutertee oder Früchtetee), das ohne Zusatz von Konservierungsstoffen, Farbstoffen und Aromastoffen hergestellt wird. Beim "Kombucha-Teepilz" handelt es sich tatsächlich nicht um einen Pilz, sondern um eine Symbiose von Essigsäurebakterien (meistens *Acetobacter xylinum,* aber auch *Acetobacter gluconicum)* mit verschiedenen Hefen (*Saccharomyces* sp., *Torula* sp., *Pichia fermentans*); fakultativ können Milchsäurebakterien vorhanden sein. Aus Gründen der Produktionssicherheit und Produktionsstabilität wird industriell gefertigter Kombucha oft wärmebehandelt.

Unter "Tee" werden erfindungsgemäß wässrige Aufgüsse von Blättern, Knospen und zarten Stielen von Thea sinensis (Camellia sinensis) und Thea assamica, aber auch von diversen anderen Pflanzen verstanden, beispielsweise Minze, Linde (Lindenblüten), Malve, Rotbusch, Kamille usw. Bevorzugte Kombucha ist aus Mono- oder Disaccharid-haltigem Tee von fermentierten (Schwarztee, Oolong-Tee, Gelber Tee, Pu-Erh-Tee) oder unfermentierten (Grüntee) Blättern von Thea sinensis (Camellia sinensis) oder Thea assamica gewonnen. Weitere bevorzugte Kombucha ist durch Fermentation von (meist mit Saccharose) gezuckertem Tee mit den symbiotischen Mikroorganismen Saccharomyces und Acetobacter xylinum gewonnen. Ein erfindungsgemäß besonders bevorzugtes Kombucha-Produkt ist ein Produkt, das durch Fermentation von mit Saccharose gezuckertem Schwarztee mit den zwei symbiotischen Mikroorganismen Saccharomyces und Acetobacter xylinum gewonnen wurde und beispielsweise unter der INCI-Bezeichnung "Saccharomyces/Xylinum/Black Tea Ferment, Glycerin, Hydroxyethylcellulose" mit der Handelsbezeichnung "Kombuchka" als Glycerin-haltige verdickte Zubereitung von der Firma Sederma erhältlich ist.

Weitere erfindungsgemäß bevorzugte Wirkstoffe, die die Kollagensynthese stimulieren, sind ausgewählt aus Olivenblattextrakten (Olea Europaea (Olive) Leaf Extract). Ein erfindungsgemäß besonders bevorzugter Olivenblattextrakt ist unter der Handelsbezeichnung Oleanoline DPG von der Firma Vincience erhältlich. Ein weiterer erfindungsgemäß besonders bevorzugter Olivenblattextrakt ist unter der Handelsbezeichnung Olea europ Fol extr. S. sicc. von der Firma Fruitarom erhältlich.

Olivenblattextrakte können einen hohen Gehalt an Oleanolsäure und/oder Oleanol aufweisen. Weitere erfindungsgemäß bevorzugte Wirkstoffe, die die Kollagensynthese stimulieren, sind ausgewählt aus Oleanolsäure und Oleanol.

Weitere erfindungsgemäß bevorzugte Wirkstoffe, die die Kollagensynthese stimulieren, sind ausgewählt aus Grüntee-Extrakten (Camellia Sinensis). Ein erfindungsgemäß besonders bevorzugter Grüntee-Extrakt ist liposomenverkapselter Grüntee-Extrakt, beispielsweise unter der Handelsbezeichnung Greenselect Phytosome von der Firma Indena erhältlich.

Weitere erfindungsgemäß bevorzugte Wirkstoffe, die die Kollagensynthese stimulieren, sind ausgewählt aus Oligopeptiden und Kreatin sowie aus Mischungen aller vorgenannten Wirkstoffe. Der oder die Wirkstoff(e), der (die) die Kollagensynthese stimuliert(en), werden in den erfindungsgemäßen Pflegeemulsionen - bezogen auf deren Gesamtgewicht - in einer Menge von 0,000001 - 5 Gew.-%, bevorzugt 0,00001 - 2 Gew.-%, besonders bevorzugt 0,0001 - 1 Gew.-% und außerordentlich bevorzugt 0,005 - 0,5 Gew.-%, eingesetzt.

Weitere erfindungsgemäß bevorzugte Pflegeemulsionen sind dadurch gekennzeichnet, dass sie zusätzlich zu der zwingenden Wirkstoffkombination nach Anspruch 1 mindestens einen weiteren, die Interaktion zwischen der extrazellulären Matrix und den Fibroblasten erhöhenden und/oder verbessernden Wirkstoff enthalten, der ausgewählt ist aus
- Apfelkernextrakten (Pyrus Malus (Apple) Fruit Extract),
- Lotuskeim-Extrakten (Nelumbo Nucifera Germ Extract),
- Extrakten aus Maiskörnern (Zea Mays (Corn) Kernel Extract),
- Rotweinextrakten,
- Traubenkernextrakten (Vitis Vinifera (Grape) Seed Extract), die bevorzugt aus der Chardonnay-Traube stammen,
- Extrakten aus Schwarzen Holunderblüten (Sambucus Nigra Flower Extract),
- Mischungen aus mindestens einem Extrakt aus Kakaobohnen (Theobroma cacao) und min destens einem Extrakt aus den Blättern der Pfefferminze (Mentha piperita),
- Hydroxystilbenen und deren Estern, insbesondere Resveratrol und/oder Resveratrolmono-, -di- und -triphosphorsäureestern und deren Salzen,
- Isoflavonoiden und Isoflavonoid-reichen Pflanzenextrakten,
- Dihydroquercetin (= Taxifolin),
- sowie Mischungen hiervon.

Weitere erfindungsgemäß bevorzugte Wirkstoffe, die die Interaktion zwischen der extrazellulären Matrix und den Fibroblasten erhöhen und/oder verbessern, sind ausgewählt aus Apfelkernextrakten (Pyrus Malus (Apple) Fruit Extract). Erfindungsgemäß besonders bevorzugte Apfelkernextrakte sind unter der Handelsbezeichnung Ederline von der Firma Seporga erhältlich. Das Produkt Ederline enthält Phytohormone, Isoflavonoide, Phytosterole, Triterpenoide, Tocopherole und natürliche Wachse. Ederline ist einmal in wasserlöslicher Form als Ederline-H (INCI: PEG-40 Hydrogenated Castor Oil, PPG-2-Ceteareth-9, Pyrus Malus (Apple) Fruit Extract), zum anderen in fettlöslicher Form als Ederline-L (INCI: Hexyldecanol, Pyrus Malus (Apple) Fruit Extract) erhältlich. Erfindungsgemäß besonders bevorzugte Pflegeemulsionen sind dadurch gekennzeichnet, dass sie den Rohstoff Ederline in Mengen von 0,1-10 Gew.-%, bevorzugt 1-8 Gew.-% und besonders bevorzugt 3-5 Gew.-%, jeweils bezogen auf die gesamte Zusammensetzung, enthalten. Erfindungsgemäß besonders bevorzugte kosmetische oder dermatologische Zusammensetzungen sind dadurch gekennzeichnet, dass sie mindestens einen Apfelkernextrakt in Mengen von 0,001 - 2 Gew.-%, bevorzugt 0,01-1,6 Gew.-% und besonders bevorzugt 0,03-1 Gew.-%, jeweils bezogen auf den Gehalt an Aktivsubstanz in der gesamten Zusammensetzung, enthalten.

Weitere erfindungsgemäß bevorzugte Wirkstoffe, die die Interaktion zwischen der extrazellulären Matrix und den Fibroblasten erhöhen und/oder verbessern, sind ausgewählt aus Lotuskeim-Extrakten. Ein erfindungsgemäß besonders bevorzugter Lotuskeim-Extrakt ist unter der Handelsbezeichnung Lotus Germ Extract mit der INCI-Bezeichnung Water, Butylene Glycol, Nelumbo Nucifera Germ Extract von der Firma Maruzen erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Extrakten aus Maiskörnern (Zea Mays (Corn) Kernel Extract). Ein erfindungsgemäß besonders bevorzugter Extrakt aus Maiskörnern ist unter der Handelsbezeichnung Deliner von der Firma Coletica erhältlich. Weitere erfindungsgemäß bevorzugte Wirkstoffe, die die Interaktion zwischen der extrazellulären Matrix und den Fibroblasten erhöhen und/oder verbessern, sind ausgewählt aus Rotweinextrakten (Wine Extract). Ein erfindungsgemäß besonders bevorzugter Rotweinextrakt ist unter der Handelsbezeichnung Sepivinol R von der Firma Seppic erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Traubenkernextrakten (Vitis Vinifera (Grape) Seed Extract), die besonders bevorzugt aus der Chardonnay-Traube stammen. Erfindungsgemäß besonders bevorzugte Traubenkernextrakte sind unter der Handelsbezeichnung Herbalia Grape von der Firma Cognis oder unter der Handelsbezeichnung Crodarom Chardonnay von Croda erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Extrakten aus Schwarzen Holunderblüten (Sambucus Nigra Flower Extract). Ein erfindungsgemäß besonders bevorzugter Extrakt aus Schwarzen Holunderblüten ist unter der Handelsbezeichnung Sambucus AO von der Firma Alpaflor/Centerchem bzw. von Permcos erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Mischungen aus mindestens einem Extrakt aus Kakaobohnen (Theobroma cacao) und mindestens einem Extrakt aus den Blättern der Pfefferminze (Mentha piperita). Erfindungsgemäß besonders bevorzugte Mischungen aus mindestens einem Extrakt aus Kakaobohnen (Theobroma cacao) und mindestens einem Extrakt aus den Blättern der Pfefferminze (Mentha piperita) - wobei wässrige, glycolische oder wässrig-glycolische Zubereitungen dieser Extraktmischungen besonders bevorzugt sind - sind unter den Handelsnamen Caomint, Caophenol, Caobromine, Caospice und Caoorange von Solabia erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Hydroxystilbenen und deren Estern, insbesondere Resveratrol (trans-Stilben-3,4'-5-triol) und/oder Resveratrolmono-, -di- und -triphosphorsäureestern und deren Salzen. Ein erfindungsgemäß besonders bevorzugter Resveratrolphosphorsäureester ist Trisodium Resveratrol Triphosphate, z. B. erhältlich von Ajinomoto.

Weitere erfindungsgemäß bevorzugte Wirkstoffe, die die Interaktion zwischen der extrazellulären Matrix und den Fibroblasten erhöhen und/oder verbessern, sind ausgewählt aus Isoflavonoiden und Isoflavonoid-reichen Pflanzenextrakten. Zu den Isoflavonoiden zählen erfindungsgemäß die Isoflavone und die Isoflavon-Glycoside.

Unter Isoflavonen sind erfindungsgemäß Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Besonders bevorzugte Isoflavone sind Daidzein, Genistein, Glycitein und Formononetin.

In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.

Weiterhin ist es erfindungsgemäß bevorzugt, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, insbesondere aus den Sojakeimen, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Lipobelle Soyaglycone (Mibelle AG Cosmetics), Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.

Erfindungsgemäß besonders bevorzugte kosmetische oder dermatologische Zusammensetzungen sind dadurch gekennzeichnet, dass sie mindestens ein Isoflavonoid in Gesamtmengen von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten kosmetischen Zusammensetzung, enthalten.

Ein weiterer erfindungsgemäß bevorzugter Wirkstoff, der die Interaktion zwischen der extrazellulären Matrix und den Fibroblasten erhöht und/oder verbessert, ist Dihydroquercetin (3,3',4',5,7-Pentahydroxyflavanon), das auch als Taxifolin bezeichnet wird.

Erfindungsgemäß besonders bevorzugte Pflegeemulsionen sind dadurch gekennzeichnet, dass sie mindestens einen Wirkstoff, der die Interaktion zwischen der extrazellulären Matrix und den Fibroblasten erhöht und/oder verbessert, in einer Gesamtmenge von 0,000001 - 10 Gew.-%, bevorzugt 0,00001 - 5 Gew.-%, besonders bevorzugt 0,0001 - 2 Gew.-% und außerordentlich bevorzugt 0,005 - 0,5 oder 1 Gew.-%, enthalten, jeweils bezogen auf den Gehalt an Aktivsubstanz in der gesamten Zusammensetzung.

Als erfindungsgemäß geeigneter Wirkstoff zur Förderung der Elastinproduktion dient ein Dillextrakt (Peucedanum Graveolens Extract), beispielsweise ein unter der Handelsbezeichnung AHYA 050 A (Gemisch aus Wasser, Butylen Glycol, Dillextrakt und Xanthan Gum) erhältliches Produkt. Dillextrakt wird in den erfindungsgemäßen Pflegeemulsionen - bezogen auf deren Gesamtgewicht - in einer Menge von 0,0001 bis 3 Gew.-%, bevorzugt von 0,0005 bis 2 Gew.-% und insbesondere 0,001 bis 1,5 Gew.-% eingesetzt.

Weitere bevorzugte Wirkstoffe, die den erfindungsgemäßen Pflegeemulsionen zusätzlich zu den zwingenden Wirkstoffen nach Anspruch zugesetzt werden können und den Hautzustand positiv beeinflussen sind ausgewählt aus der Gruppe, die gebildet wird aus:
- DNA- oder RNA-Oligonucleotiden,
- Vitaminen, Provitaminen und Vitaminvorstufen der Gruppen B, E, H und K und den Estern der vorgenannten Substanzen,
- α-Hydroxycarbonsäuren, α-Ketocarbonsäuren, β-Hydroxycarbonsäuren und deren Ester-, Lacton- und/oder Salzform,
- Flavonoiden und Flavonoid-reichen Pflanzenextrakten,
- Ubichinon und Ubichinol sowie deren Derivaten,
- Silymarin,
- Ectoin,
- anorganischen und organischen UV-Filtersubstanzen,
- selbstbräunenden,
- hautberuhigenden und
- sebumregulierenden Wirkstoffen sowie
- Mischungen dieser Wirkstoffe.

Weitere erfindungsgemäß bevorzugte Pflegeemulsionen sind dadurch gekennzeichnet, dass sie zusätzlich zu der zwingenden Wirkstoffkombination nach Anspruch 1 mindestens einen Wirkstoff enthalten, der ausgewählt ist aus DNA-Oligonucleotiden und RNA-Oligonucleotiden. Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glycosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5 '-triphosphat) und GTP (Guanosin-5'-triphosphat).

Ein erfindungsgemäß besonders bevorzugtes Oligonucleotid ist das Thymidin-Dinucleotid.

In den besonders bevorzugten erfindungsgemäßen Pflegeemulsionen sind die DNA-Oligonucleotide oder RNA-Oligonucleotide in Mengen von 0,00001-5 Gew.-%, bevorzugt 0,0001 - 1,0 Gew.-% und besonders bevorzugt 0,0005-0,5 Gew.-%, bezogen auf die gesamte Zusammensetzung, enthalten.

Weitere erfindungsgemäß bevorzugte Pflegeemulsionen sind dadurch gekennzeichnet, dass sie zusätzlich zu der zwingenden Wirkstoffkombination nach Anspruch 1 mindestens einen Wirkstoff enthalten, der ausgewählt ist aus mindestens einem Vitamin, Provitamin oder einer als Vitaminvorstufe bezeichnete Verbindung aus den Vitamingruppen B, E, H und K und den Estern der vorgenannten Substanzen.

Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf die gesamte Zusammensetzung, eingesetzt.
- Vitamin B₂, Trivialname Riboflavin, chemische Bezeichung 7,8-Dimethyl-10-(1-D-ribityl)-benzo[g]pteridin-2,4(3*H*,10*H*)-dion. Bevorzugt werden Riboflavin oder seine Derivate in Mengen von 0,05 bis 1 Gew.-%, bezogen auf die gesamte Zusammensetzung, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Zusammensetzungen bevorzugt in Mengen von 0,05 bis 1 Gew.-%, bezogen auf die gesamte Zusammensetzung, enthalten ist.
- Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführ ungsform der Erfindung können an Stelle von sowie zusätzlich zu Pantothensäure oder Pan thenol auch Derivate des 2-Furanon mit der allgemeinen Strukturformel (VIT-I) eingesetzt werden.

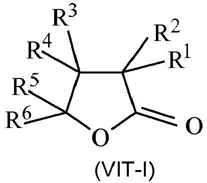

Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄- Kohlenwasserstoffrest, einen gesättigten oder ein- oder zweifach ungesättigten, verzweigten oder linearen Mono-, Di- oder Trihydroxy-C₂-C₄- Kohlenwasserstoffrest oder einen gesättigten oder ein- oder zweifach ungesättigten, verzweigten oder linearen Mono-, Di- oder Triamino-C₂-C₄- Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-y-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (VIT-I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.

Die genannten Verbindungen des Vitamin B₅-Typs sowie die 2-Furanonderivate sind in den erfindungsgemäßen Mitteln in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin B₆ ist in den erfindungsgemäßen Mitteln bevorzugt in Mengen von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3aS,4S, 6a*R*)-2-Oxohexahydrothienol[3,4-*d*]-imidazol-4-valeriansäure. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten.

- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind dadurch gekennzeichnet, dass sie mindestens eine Komponente, ausgewählt aus Orotsäure, Orotaten und deren Estern, in einer Gesamtmenge von 0,0001-1,0 Gew.-%, insbesondere 0,01 - 0,5 Gew.-%, bezogen auf die Zusammensetzung, enthalten.

Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, - succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05-1 Gew.-%, bezogen auf die gesamte Zusammensetzung, enthalten.

Vitamin H ist eine andere Bezeichnung für Biotin oder Vitamin B₇ (siehe oben).

Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.-%, jeweils bezogen auf die gesamte Zusammensetzung, enthalten.

Weitere erfindungsgemäß bevorzugte Pflegeemulsionen sind dadurch gekennzeichnet, dass sie zusätzlich zu der zwingenden Wirkstoffkombination nach Anspruch 1 mindestens einen Wirkstoff enthalten, der ausgewählt ist aus mindestens einer α-Hydroxycarbonsäure, α-Ketocarbonsäure oder β-Hydroxycarbonsäure oder deren Ester-, Lacton- und/ oder Salzform. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Eine besonders bevorzugte α-Ketocarbonsäure ist Brenztraubensäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Weitere Derivate der vorgenannten Säuren sind deren physiologisch verträglichen Salze, bevorzugt die Zink-, Kupfer- und Mangansalze, die Salze der Alkali- und der Erdalkalimetalle und die Ammonium-, Alkylammonium-, Alkanolammonium- und Glucammoniumsalze, besonders bevorzugt die Natrium-, Kalium-, Magnesium- und CalciumSalze.

Die α-Hydroxycarbonsäuren, α-Ketocarbonsäuren oder β-Hydroxycarbonsäuren oder ihre Derivate sind bevorzugt in einer Gesamtmenge von 0,1-10 Gew.-%, besonders bevorzugt 0,5 - 5 Gew.-% und außerordentlich bevorzugt 1 - 2 Gew.-%, jeweils bezogen auf die gesamte Zusammensetzung, enthalten.

Weitere erfindungsgemäß bevorzugte Pflegeemulsionen sind dadurch gekennzeichnet, dass sie zusätzlich zu der zwingenden Wirkstoffkombination nach Anspruch 1 mindestens einen Wirkstoff enthalten, der ausgewählt ist aus mindestens einem Flavonoid und/oder mindestens einem Flavonoid-reichen Pflanzenextrakt.

Die erfindungsgemäß bevorzugten Flavonoide umfassen die Glycoside der Flavone, der 3-Hydroxyflavone (Flavonole) und der Aurone. Die Flavanone und die Isoflavone sind erfindungsgemäß ausdrücklich von den Flavonoiden ausgenommen. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin(3,3',4',5-Tetrahydroxy-7-(2-hydroxyethoxy)flavon-3-(6-O-(6-deoxy-α-L-mannopyranosyl)-β-D-glucopyranosid)), Eriodictin und Apigenin-7-glucosid (4',5,7-Trihydroxyflavon-7-glucosid).

Erfindungsgemäß außerordentlich bevorzugte Flavonoide sind α-Glucosylrutin, Naringin und Apigenin-7-glucosid.

Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin und Neohesperidindihydrochalkon.

Erfindungsgemäß werden die Flavonoide in Mengen von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, eingesetzt.

Weitere erfindungsgemäß bevorzugte Pflegeemulsionen sind dadurch gekennzeichnet, dass sie zusätzlich zu der zwingenden Wirkstoffkombination nach Anspruch 1 mindestens einen Wirkstoff enthalten, der ausgewählt ist aus mindestens einem Ubichinon oder einem Ubichinol oder deren Derivaten. Ubichinole sind die reduzierte Form der Ubichinone. Die erfindungsgemäß bevorzugten Ubichinone weisen die Formel (UBI-I) auf: 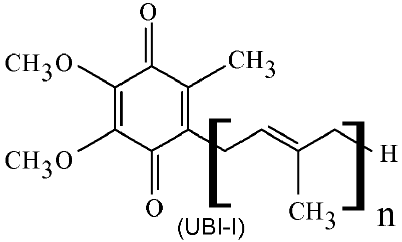 mit n = 6, 7, 8, 9 oder 10.

Besonders bevorzugt ist das Ubichinon der Formel (UBI-I) mit n = 10, bekannt als Coenzym Q10. Erfindungsgemäß werden die Ubichinone, Ubichinole oder deren Derivate in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt.

Weitere erfindungsgemäß bevorzugte Pflegeemulsionen sind dadurch gekennzeichnet, dass sie zusätzlich zu der zwingenden Wirkstoffkombination nach Anspruch 1 Silymarin enthalten. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören.

Erfindungsgemäß wird Silymarin in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt.

Weitere erfindungsgemäß bevorzugte Pflegeemulsionen sind dadurch gekennzeichnet, dass sie zusätzlich zu der zwingenden Wirkstoffkombination nach Anspruch 1 Ectoin enthalten. Ectoin ist der Trivialname für 2-Methyl-1,4,5,6-tetrahydropyrimidin-4-carboxylat. Erfindungsgemäß ist Ectoin bevorzugt in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

Weitere erfindungsgemäß bevorzugte Pflegeemulsionen sind dadurch gekennzeichnet, dass sie zusätzlich zu der zwingenden Wirkstoffkombination nach Anspruch 1 mindestens eine anorganische und/oder mindestens eine organische UV-Filtersubstanz enthalten.

Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt.

Die erfindungsgemäß bevorzugten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Die Benzoxazol-Derivate liegen vorteilhaft in gelöster Form in den erfindungsgemäßen kosmetischen Zubereitungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen. Erfindungsgemäß besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 2-(4'-Diethylamino-2'-hydroxybenzoyl)-benzoesäurehexylester (auch: Aminobenzophenon, unter der Bezeichnung Uvinul A Plus bei der Firma BASF erhältlich), 4-Methoxybenzmalonsäuredi-2-ethylhexylester, an Polymere gebundene UV-Filter, z. B. das 3-(4-(2,2-Bis-Ethoxycarbonylvinyl)-phenoxy)propenyl)-methoxysiloxan/ Dimethylsiloxan-Copolymer mit der INCI-Bezeichnung Dimethicodiethylbenzal malonate (CAS-Nr. 207574-74-1, Parsol^{®} SLX), Triazinderivate, wie z. B. 2,4-Bis-{[4-(2-Ethylhexyloxy)-2-hydroxy]-phenyl}-6-(4-methoxyphenyl)-1,3,5-triazin (INCI: Bis-Ethylhexyloxyphenol Methoxyphenyl Triazin, unter dem Namen Tinosorb S bei CIBA erhältlich), Dioctylbutylamidotriazon (INCI: Diethylhexyl Butamido Triazone, unter dem Namen Uvasorb^{®} HEB bei Sigma 3V erhältlich), 2,4,6-Trianilino-(p-carbo-2'-ethyl-1'-hexyloxy)-1,3,5-triazin (Ethylhexyl Triazone, Uvinul^{®} T 150), 2-[4,6-Bis(2,4-dimethylphenyl)-1,3,5-triazin-2-yl]-5-(octyloxy)phenol (CAS Nr.: 2725-22-6), 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma), die Benzotriazolderivate 2,2'-Methylen-bis-(6-(2H-benzotriazol-2-yl)-4-(1,1,3,3-tetramethylbutyl)-phenol) [Tinosorb M (Ciba)], 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4), 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane, 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin oder auch Aniso Triazin, erhältlich als Tinosorb^{®} S von CIBA), 2,4-Bis-{[4-(3-sulfonato)-2-hydroxy-propyloxy)-2-hydroxy]-phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethylcarboxyl)-phenylamino]-1,3,5-triazin, 2,4-Bis-{[4-(3-(2-propyloxy)-2-hydroxy-propyloxy)-2-hydroxy]-phenyl}-6-[4-(ethylcarboxyl)-phenylamino]-1,3,5-triazin, 2,4-Bis-{[4-(2-ethylhexyloxy)-2-hydroxy]-phenyl}-6-(1-methyl-pyrrol-2-yl)-1,3,5-triazin,2,4-Bis-{[4-tris(trimethylsiloxy-silylpropyloxy)-2-hydroxy]-phenyl}-6-(4-methoxyphenyl)-1,3,5-triazin, 2,4-Bis-{[4-(2-methylpropenyloxy)-2-hydroxy]-phenyl}-6-(4-methoxyphenyl)-1,3,5-triazin,2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin sowie Mischungen der genannten Komponenten.

Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, insbesondere die Sulfonsäure selbst mit der INCI Bezeichnung Phenylbenzimidazole Sulfonic Acid (CAS.-Nr. 27503-81-7), die beispielsweise unter dem Handelsnamen Eusolex 232 bei Merck oder unter Neo Heliopan Hydro bei Symrise erhältlich ist, und das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonate (CAS-Nr.: 180898-37-7), das beispielsweise unter dem Handelsnamen Neo Heliopan AP bei Symrise erhältlich ist, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der INCI-Bezeichnung Terephthalydene Dicampher Sulfonic Acid (CAS.-Nr.: 90457-82-2, als Mexoryl SX von der Firma Chimex erhältlich).

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.

Erfindungsgemäß sind die organischen UV-Filtersubstanzen bevorzugt in Mengen von 0,1 - 30 Gew.-%, besonders bevorzugt 0,5 - 20 Gew.-%, außerordentlich bevorzugt 1,0-15 Gew.-% und weiter bevorzugt 3,0-10 Gew.-%, jeweils bezogen auf die gesamte Zusammensetzung, enthalten.

Erfindungsgemäß sind die anorganischen UV-Filtersubstanzen bevorzugt in Mengen von 0,1-15 Gew.-%, mehr bevorzugt 0,5-10 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten. Weitere erfindungsgemäß bevorzugte Pflegeemulsionen sind dadurch gekennzeichnet, dass sie zusätzlich zu der zwingenden Wirkstoffkombination nach Anspruch 1 mindestens einen selbstbräunenden Wirkstoff enthalten. Erfindungsgemäß bevorzugte selbstbräunende Wirkstoffe sind ausgewählt aus Dihydroxyaceton, Erythrulose und 5,6-Dihydroxyindolin.

Erfindungsgemäß sind die selbstbräunenden Wirkstoffe in einer Gesamtmenge von 0,01-15 Gew.-%, bevorzugt 0,1-10 Gew.-%, besonders bevorzugt 1,0-5 Gew.-% und außerordentlich bevorzugt 2,0 - 4,0 Gew.-%, jeweils bezogen auf die gesamte Zusammensetzung, enthalten. Weitere erfindungsgemäß bevorzugte Pflegeemulsionen sind dadurch gekennzeichnet, dass sie zusätzlich zu der zwingenden Wirkstoffkombination nach Anspruch 1 mindestens einen hautberuhigenden Wirkstoff enthalten. Erfindungsgemäß bevorzugte hautberuhigende Wirkstoffe sind ausgewählt aus Allantoin, α-Bisabolol, α-Liponsäure, Extrakten aus Centella asiatica, beispielsweise erhältlich unter der Bezeichnung Madecassicoside von DSM, Glycyrrethinsäure, die besonders bevorzugt in Liposomen verkapselt vorliegt und in dieser Form z. B. unter dem Handelsnamen Calmsphere von Soliance erhältlich ist, Mischungen aus Getreidewachsen, Extrakten aus Schibutter und Argania Spinosa-Öl mit der INCI-Bezeichnung "Spent grain wax and Butyrospermum Parkii (shea butter) extract and Argania Spinosa Kernel Oil", wie sie z. B. unter der Handelsbezeichnung Stimu-Tex AS von der Firma Pentapharm erhältlich sind, Extrakten aus Vanilla Tahitensis, wie sie z. B. unter der Handelsbezeichnung Vanirea (INCI : Vanilla Tahitensis Fruit Extract) von der Firma Solabia erhältlich sind, Alginhydrolysaten, wie sie z. B. unter der Handelsbezeichnung Phycosaccharide, insbesondere Phycosaccharide AI, von der Firma Codif erhältlich sind, Extrakten aus Bacopa Monniera, wie sie z. B. unter der Handelsbezeichnung Bacocalmine von der Firma Sederma erhältlich sind, Extrakten aus der Rooibos-Pflanze, wie sie z. B. unter dem Handelsnamen Rooibos Herbasec MPE von der Firma Cosmetochem erhältlich sind, Hefeextrakten, besonders bevorzugt das Handelsprodukt Drieline (INCI-Bezeichnung "Sorbitol, Yeast Extract"), erhältlich von der Firma Lanatech, den physiologisch verträglichen Salzen von Sterolsulfaten, wie sie z. B. unter dem Handelsnamen Phytocohesine (INCI: Sodium Beta-Sitosterylsulfate) von der Firma Vincience erhältlich sind, Aminodicarbonsäuren mit einer C-Kettenlänge von 3 - 6 Kohlenstoffatomen sowie deren physiologisch verträglichen Salzen, bevorzugt ausgewählt aus Aminomalonsäure, Aminobernsteinsäure (= Asparaginsäure), Aminoglutarsäure und Aminoadipinsäure sowie deren physiologisch verträglichen Salzen wie Kaliumaspartat und Magnesiumaspartat, Extrakten aus den Samen von Cucurbita pepo (Zucchini), Extrakten aus Mentha piperita, insbesondere aus den Blättern von Mentha piperita, N-acylierten Alkanolaminen, insbesondere N-Palmitoylethanolamin, Hydrolysaten und/oder Extrakten aus der Alge *Enteromorpha compressa* (Darmtang, eine Grünalge), insbesondere aus dem Vegetationskörper (Thallus) der Alge, kommerziell erhältlich z. B. unter dem Handelsnamen Enteline 2, weiterhin ausgewählt aus dem Dipeptid Tyr-Arg, dessen Estern und dessen N-AcylDerivaten, insbesondere N-Acetyl-Tyr-Arg-hexyldecylester (z. B. Calmosensine von Sederma, sowie beliebigen Mischungen dieser Substanzen.

Erfindungsgemäß besonders bevorzugte Extrakte aus den Samen von Cucurbita pepo (Zucchini) sind beispielsweise in den Handelsprodukten Curbilene (ex Indena SpA, INCI: Cucurbita pepo (pumpkin) seed extract), Ocaline (ex Soliance, INCI: Sea Water (and) Water (and) Cucurbita pepo (pumpkin) seed extract), ARP 100 (ex Greentech, INCI: Water, Alcohol, Serenoa Serrulata Fruit Extract, Epilobium Angustifolium Extract, Cucurbita Pepo (Pumpkin) Seed Extract), ARP 100 Huileux (ex Greentech, INCI: Caprylic/Capric Triglyceride, Serenoa Serrulata Fruit Extract, Epilobium Angustifolium Flower/Leaf/Stem Extract, Cucurbita Pepo (Pumpkin) Seed Extract), Cucurbitine (ex Christian Dior Parfums, INCI: Water (and) Cucurbita pepo (pumpkin) seed extract), Herbasol Extract Pumpkin (ex Cosmetochem, INCI: Water (and) Propylene glycol (and) Cucurbita pepo (pumpkin) seed extract) und Pumpkin Extract (ex Draco Natural Products, INCI: Water (and) Cucurbita pepo (pumpkin) seed extract) enthalten.

Erfindungsgemäß besonders bevorzugte Extrakte aus Mentha piperita, insbesondere aus den Blättern von Mentha piperita, sind beispielsweise in den Handelsprodukten Calmiskin OP (ex Silab, INCI: Water (and) Mentha piperita (Peppermint) Leaf Extract) und Caomint (ex Solabia, INCI: Propylene Glycol, Water, Mentha piperita (Peppermint) Leaf Extract, Theobroma Cacao (Cocoa) Extract) enthalten.

Die hautberuhigenden Wirkstoffe sind bevorzugt in Mengen von 0,001 bis 5 Gew.-%, besonders bevorzugt 0,01 bis 2 Gew.-% und außerordentlich bevorzugt 0,1 bis 1 Gew.-%, jeweils bezogen auf die gesamte Zusammensetzung, enthalten.

Weitere erfindungsgemäß bevorzugte Pflegeemulsionen sind dadurch gekennzeichnet, dass sie zusätzlich zu der zwingenden Wirkstoffkombination nach Anspruch 1 mindestens einen sebumregulierenden Wirkstoff. Erfindungsgemäß bevorzugte sebumregulierende Wirkstoffe sind ausgewählt aus Azelainsäure, Sebacinsäure, 10-Hydroxydecansäure, 1,10-Decandiol, die als erfindungsgemäß besonders bevorzugte Dreierkombination in dem Handelsprodukt Acnacidol PG von Vincience eingesetzt werden, weiterhin aus dem Handelsprodukt Azeloglicina (Potassium Azeloyl Diglycinate) von Sinerga, Extrakten aus Spiraea Ulmaria, wie sie z. B. im Produkt Seboregul der Firma Silab enthalten sind, weiterhin aus wasser- und öllöslichen Extrakten aus Hamamelis, Klettenwurzel und Brennessel, Zimtbaumextract (z. B. Sepicontrol^{®} A5 von der Firma Seppic), Chrysanthemenextrakt (z. B. Laricyl^{®} von Laboratoires Serobiologiques) und den Wirkstoffmischungen Asebiol^{®} BT 2 (Laboratoires Serobiologiques, INCI: Aqua, Hydrolyzed Yeast Protein, Pyridoxine, Niacinamide, Glycerin, Panthenol, , Allantoin, Biotin) und Antifettfaktor^{®} COS-218/2-A (Cosmetochem, INCI: Aqua, Cetyl-PCA, PEG-8 Isolauryl Thioether, PCA, Cetyl Alcohol). Die sebumregulierenden Wirkstoffe sind bevorzugt in einer Gesamtmenge von 0,001 bis 5 Gew.-%, besonders bevorzugt 0,01 bis 2 Gew.-% und außerordentlich bevorzugt 0,1 bis 1 Gew.-%, jeweils bezogen auf die gesamte Zusammensetzung, enthalten.

Weitere erfindungsgemäß bevorzugte Pflegeemulsionen sind dadurch gekennzeichnet, dass sie zusätzlich zu der zwingenden Wirkstoffkombination nach Anspruch 1 mindestens ein Polysaccharid enthalten, das in einer bevorzugten Ausführungsform ausgewählt ist aus Glycosaminglycanen, besonders bevorzugt aus Hyaluronsäure, Dextran, Dextransulfat, Chondroitin-4-sulfat und Chondroitin-6-sulfat und insbesondere bevorzugt aus Hyaluronsäure. Das Polysaccharid wird den erfindungsgemäßen Pflegeemulsionen - bezogen auf deren Gesmtgewicht - in einer Menge von 0,0001 bis 2 Gew.-%, bevorzugt von 0,0005 bis 1,5 Gew.-% und insbesondere in Mengen von 0,001 bis 1 Gew.-% zugegeben.

In einer besonders bevorzugten Ausführungsform der Erfindung enthalten die Pflegeemulsionen zusätzlich zu der zwingenden Wirkstoffkombination nach Anspruch 1 mindestens ein Feuchthaltemittel bzw. einen Penetrationshilfsstoff. Hierzu sind beispielsweise zu zählen Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Sorbitol, Milchsäure, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol. Besonders bevorzugt sind Glycerin und Sorbitol.

Die Feuchthaltemittel bzw. Penetrationshilfsstoffe werden den erfindungsgemäßen Pflegeemulsionen - bezogen auf deren Gesamtgewicht - in einer Menge von 0,1 bis 15 Gew.-%, bevorzugt von 0,25 bis 10 Gew.-% und insbesondere von 0,5 bis 5 Gew.-% zugesetzt.

Die erfindungsgemäßen Pflegeemulsionen weisen einen pH-Wert im hautschonenden Bereich von 3,5 bis 7,5, bevorzugt von 4 bis 7 und insbesondere von 4,5 bis 6,5 auf.

Darüber hinaus können die erfindungsgemäßen Emulsionen fakultativ die weiteren Hilfs-, Zusatz- und Wirkstoffe enthalten:
- Desodorierende Wirkstoffe wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker.
- Insekten-Repellentien: Als Insekten-Repellentien kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent 3535 von der Merck KGaA vertrieben wird, sowie Butylacetylaminopropionate in Frage.
- Selbstbräuner: Als Selbstbräuner eignet sich Dihydroxyaceton. Als Tyrosininhibitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.
- Konservierungsmittel: Als Konservierungsmittel eignen sich beispielsweise Phenoxyethanol, Formaldehydlösung, Parabene, Pentandiol oder Sorbinsäure sowie die unter der Bezeichnung SurfacineG bekannten Silberkomplexe und die in Anlage 6, Teil A und B der Kosmetikverordnung aufgeführten weiteren Stoffklassen.
- Parfümöle: Als Parfümöle seien genannt Gemische aus natürlichen und synthetischen Riechstoffen. Natürliche Riechstoffe sind Extrakte von Blüten, Stängeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.
- Farbstoffe: Als Farbstoffe können die für kosmetische Zwecke geeigneten und zugelassenen Substanzen verwendet werden. Beispiele sind Cochenillerot A (C.I. 16255), Patentblau V (C.1.42051), Indigotin (C.1.73015), Chlorophyllin (C.1.75810), Chinolingelb (C.1.47005), Titandioxid (C.1. 77891), Indanthrenblau RS (C.1. 69800) und Krapplack (C.1.58000). Diese Farbstoffe werden üblicherweise in Konzentrationen von 0,001 bis 0,1 Gew.-%, bezogen auf die gesamte Mischung, eingesetzt.

Die erfindungsgemäßen Pflegeemulsionen sind insbesondere für die Applikation in einem manuell betätigbaren System mit Pumpmechanismus geeignet.

Besonders bevorzugt ist es, wenn die erfindungsgemäße Pflegeemulsion in einem Schaumspender mit einem Pumpmechanismus zur Verschäumung mit Luft enthalten ist. Ein derartiger Schaumspender ermöglicht es, auf Treibgase zu verzichten. Besonders vorteilhaft bei diesem Typ von Spendern ist auch, dass die Anwendung besonders einfach ist, der entstehende Schaum eine leichte und cremige Textur, keine Drainage sowie eine einfache Verteilbarkeit aufweist, wodurch alle vorgenannten positiven Effekte auf der Haut erzielt werden können. Erfindungsgemäß besonders geeignete Schaumspender werden in den Anmeldungen WO 2003/088941 und WO 2004/078902 ausführlich beschrieben. Auf diese Anmeldungen wird hinsichtlich der Beschreibung des Pumpspenders daher ausdrücklich Bezug genommen.

Diese für die erfindungsgemäßen Pflegeemulsionen besonders geeigneten Schaumspender sind im Handel unter den Bezeichnungen M3 und F3 von der Firma Airspray erhältlich.

Um unter Verwendung eines manuellen Pumpspenders aufschäumbar zu sein, sind bevorzugte erfindungsgemäße Zusammensetzungen gekennzeichnet durch eine Viskosität bei 21 °C im Bereich von 50 - 1500 mPas, bevorzugt 100 - 1000 mPas, besonders bevorzugt 400-850 mPas, außerordentlich bevorzugt 500-750 mPas, gemessen mit einem Brookfield Rotationsviskosimeter, Spindel Nr. 1 bei einer Rotationsgeschwindigkeit von 20 UpM oder mit einer Spindel T-A bei einer Rotationsgeschwindigkeit von 12 UpM.

Die folgenden Beispiele sollen den Gegenstand der Erfindung näher beschreiben, ohne ihn darauf einzuschränken. Die Mengenangaben beziehen sich dabei - sofern nicht anders angegeben - auf Gew.-%.

### Beispiel für eine schäumbare Sonnenschutzemulsion

| **Handelsname** | **INCI-Bezeichnung und Hersteller** | **Menge** |
|---|---|---|
| **Dehymuls® PGPH** | Polyglyceryl-2-Dipopyhydroxystearate (Cognis) | 2,5 |
| **ABIL® EM 90** | Cetyl PEG/PPG-10/1-Dimethicone (Evonik Degussa) | 2,5 |
| **Cetiol® OE** | Dicaprylyl Ether (Cognis) | 1,0 |
| **Cegesoft® C24** | Ethylhexyl Palmitate (Cognis) | 3,0 |
| **Finsolv® TN** | C12-C15-Alkylbenzoat (Innospec) | 3,0 |
| **Parsol® SLX** | Benzylidene Malonate Polysiloxane (DSM) | 3,0 |
| **Neo Heliopan® 303** | Octocrylene (Symrise) | 4,0 |
| **Parsol® 1789** | Butyl Methoxydibenzoylmethane (DSM) | 2,0 |
| **Tocopheryl Acetat** | Tocopheryl Acetat | 0,6 |
| **Sepinov® EMT 10** | Hydroxyethyl Acrylate/Sodium - Acryloyldimethyl Taurate Copolymer (Seppic) | 0,1 |
| **Sorbit, 70%** | Sorbit | 1,5 |
| **Glycerin, 86%** | Glycerin | 1,5 |
| **Tego Carbomer® 140** | Carbomer (Goldschmidt) | 0,12 |
| **Protanal Ester® CF** | Propylene Glycol Alginate (FMC Biopolymers) | 0,4 |
| **Parfum** | | 0,3 |
| **Ridulisse® C GR** | Hydrolized Soy Protein (Silab) | 0,5 |
| **Phenonip® ME** | Phenoxyethanol, Methylparaben, Ethylparaben (Clariant) | 1,0 |
| **Texapon® SB 3 unk.** | Disodium Laureth Sulfosuccinate (Cognis) | 3,0 |
| **Tego Betain® 810** | Capryl/Capramidopropyl Betaine (Goldschmidt) | 2,0 |
| **Citronensäure, 10%ig** | Citronensäure | q.s. |

Die Fett- und die Wasserphase werden auf ca. 80°C erhitzt. Anschließend wird die Wasserphase unter Verwendung einer Dissolverscheibe mit der vorgelegten Fettphase emulgiert. Die Rührerdrehfrequenz wird während der Phasenvereinigung kontinuierlich von 250 auf 1400 rpm gesteigert. Bei einer Temperatur von 60°C bzw. 40°C werden die Polymere (Carbomer und Alginatester) in Form einer Quellung in Wasser hinzugegeben. Nachdem die Emulsion unter Rühren auf ca. 30°C abgekühlt ist, werden die Duftstoffe, Wirkstoffe und Konservierungsmittel eingearbeitet. Im Anschluss wird zuerst ein Drittel der hydrophilen Emulgatormischung hinzugegeben. Bei vollständiger Durchmischung nimmt die Viskosität der Emulsion stark ab und die Drehfrequenz des Rührorgans wird auf 300 rpm herunter geregelt. Direkt danach werden die verbleibenden zwei Drittel der hydrophilen Emulgatormischung eingearbeitet und die Drehfrequenz auf unter 300 rpm gesenkt. Der pH-Wert wird auf 5,1 eingestellt. Die Emulsion wird in einem Foamer des Modells F3 oder M3 der Firma Airspray konfektioniert. Der jeweilige Pflegeschaum kann auf diese Weise einfach und in der richtigen Dosierung für die Anwendung entnommen werden. Der Schaum ist besonders cremig und hinterlässt nach seiner Absorption auf der Haut ein mit Feuchtigkeit gesättigtes, glattes Hautgefühl.

## Patentansprüche

1. Kosmetische Pflegeemulsion, die unter Verwendung eines manuellen Pumpspenders aufschäumbar ist, enthaltend
(A) eine Emulgatorkombination (i) + (ii) aus mindestens zwei W/O-Emulgatoren gleichen HLB-Werts, ausgewählt aus
(i) mindestens einem Poly(12-hydroxystearinsäure)polyglycerinester und
(ii) mindestens einem Silikonemulgator sowie
(B) eine Tensidkombination (iii) + (iv) aus
(iii) mindestens einem anionischen Tensid und
(iv) mindestens einem amphoteren und/oder zwitterionischen Tensid, und
(C) 1 - 25 Gew.-%, bezogen auf das Gesamtgewicht der Pflegeemulsion, mindestens einer Öl- oder Fettkomponente.

2. Pflegeemulsion nach Anspruch 1, **dadurch gekennzeichnet, dass** die HLB-Werte der Emulgatoren im Bereich von 2 bis 8, bevorzugt im Bereich von 3 bis 7 und insbesondere im Bereich von 4 bis 6 liegen.

3. Pflegeemulsion nach einem der Ansprüche 1 oder 2, **dadurch gekennzeichnet, dass** sie die Emulgatoren (i) und (ii) in einem Gewichtsverhältnis von 5 : 1 bis 1 : 4, bevorzugt von 3 : 1 bis 1 : 2 und insbesondere von 2 : 1 bis 1 : 1 enthält.

4. Pflegeemulsion nach einem der Ansprüche 1 bis 3, **dadurch gekennzeichnet, dass** sie als Silikonemulgator ein Organopolysiloxan-Polyoxyalkylen enthält.

5. Pflegeemulsion nach Anspruch 4, **dadurch gekennzeichnet, dass** der Silikonemulgator eine C8-C18-Alkylgruppe sowie eine oder mehrere Ethoxy- und/oder Propoxy-Gruppen enthält.

6. Pflegeemulsion nach einem der Ansprüche 1 bis 5, **dadurch gekennzeichnet, dass** sie als Tensidkombination eine Mischung aus milden anionischen und milden amphoteren und/oder zwitterionischen Tensiden enthält.

7. Pflegeemulsion nach Anspruch 6, **dadurch gekennzeichnet, dass** die Tenside ausgewählt sind aus einem oder mehreren Tensiden der Gruppe (iii) der Acylaminosäuren, alpha-Olefinsulfonate, ethoxylierte Alkylschwefelsäurester, Sulfobernsteinsäureester, Ethercarbonsäuren und/oder den Salzen dieser Verbindungen, sowie aus einem oder mehreren Tensiden der Gruppe (iv) der Alkylbetaine, Alkylamidoalkylbetaine, Aminopropionate, Aminoglycinate, Sulfobetaine und/oder Imidazoliniumbetaine und/oder den Salzen dieser Verbindungen.

8. Pflegeemulsion nach einem der Ansprüche 1 bis 7, **dadurch gekennzeichnet, dass** als Öl- oder Fettkomponente eine Kombination aus Dicaprylylether, Ethylhexylpalmitat und C₁₂-C₁₅-Alkylbenzoat enthalten ist.

9. Pflegeemulsion nach einem der Ansprüche 1 bis 8, **dadurch gekennzeichnet, dass** sie zur Erhöhung der Emulsions- und Schaumstabilität eine Kombination aus mindestens zwei Polymeren enthält, von denen eines natürlichen und das andere synthetischen Ursprungs ist.

10. Pflegeemulsion nach Anspruch 9, **dadurch gekennzeichnet, dass** die Polymere synthetischen Ursprungs ausgewählt sind aus Polyacrylsäuren und/oder Polyacryloyldimethyltauraten und die Polymere natürlichen Ursprungs ausgewählt sind aus Xanthan Gum-, Guar Gum-, Alginat- und/oder Carrageenat-Derivaten.

11. Pflegemulsion nach einem der Ansprüche 1 bis 10, **dadurch gekennzeichnet, dass** sie eine Kombination verschiedener Emulsionssysteme umfasst.

12. Pflegeemulsion nach einem der Ansprüche 1 bis 11, **dadurch gekennzeichnet, dass** sie mindestens einen weiteren kosmetischen Hilfs- oder Wirkstoff enthält, der ausgewählt ist aus der Gruppe der Sonnenschutzmittel, Feuchthaltemittel, Antioxidantien, Hyaluronsäure sowie deren physiologisch verträglichen Salzen und Derivaten, Wirkstoffen zur Stimulierung der Kollagensynthese, Wirkstoffen zur Erhöhung bzw. Verbesserung der Interaktion zwischen der extrazellulären Matrix und den Fibroblasten sowie Wirkstoffen zur Förderung der Elastinproduktion.

13. Pflegeemulsion nach einem der Ansprüche 1 bis 12, **dadurch gekennzeichnet, dass** sie einen pH-Wert im Bereich von 3,5 bis 7,5, bevorzugt von 4 bis 7 und insbesondere von 4,5 bis 6,5 aufweist.

14. Verwendung einer Mischung aus einer Emulgatorkombination (A), enthaltend zwei W/O-Emulgatoren gleichen HLB-Werts, die ausgewählt sind aus
(i) mindestens einem Poly(12-hydroxystearinsäure)polyglycerinester und
(ii) mindestens einem Silikonemulgator,
einer Tensidkombination (B), enthaltend
(iii) mindestens ein anionisches Tensid und
(iv) mindestens ein amphoteres und/oder zwitterionisches Tensid, und
1 - 25 Gew.-% mindestens einer Öl- oder Fettkomponente, bezogen auf das Gesamtgewicht der Pflegeemulsion, zur Herstellung kosmetischer Pflegeemulsionen, die unter Verwendung eines manuellen Pumpspenders aufschäumbar sind und eine leichte Textur sowie eine bessere Verteilbarkeit aufweisen.

15. Verwendung einer Pflegeemulsion nach einem der Ansprüche 1 bis 13 zur Verbesserung des Hautgefühls.

## Claims

1. A cosmetic care emulsion that can be foamed using a manual pump dispenser, containing
(A) an emulsifier combination (i) + (ii) of at least two W/O emulsifiers of the same HLB value, selected from
(i) at least one poly(12-hydroxystearic acid)polyglycerol ester and
(ii) at least one silicone emulsifier, and
(B) a surfactant combination (iii) + (iv) of
(iii) at least one anionic surfactant and
(iv) at least one amphoteric and/or zwitterionic surfactant, and
(C) 1-25 wt.%, based on the total weight of the care emulsion, of at least one oil or fat component.

2. The care emulsion according to claim 1, **characterized in that** the HLB values of the emulsifiers are in the range of from 2 to 8, preferably in the range of from 3 to 7, and in particular in the range of from 4 to 6.

3. The care emulsion according to either claim 1 or claim 2, **characterized in that** it contains the emulsifiers (i) and (ii) in a weight ratio of 5 : 1 to 1 : 4, preferably 3 : 1 to 1 : 2, and particularly 2 : 1 to 1 : 1.

4. The care emulsion according to one of claims 1 to 3, **characterized in that** it contains an organopolysiloxane-polyoxyalkylene as the silicone emulsifier.

5. The care emulsion according to claim 4, **characterized in that** the silicone emulsifier contains a C8-C18 alkyl group and one or more ethoxy and/or propoxy groups.

6. The care emulsion according to one of claims 1 to 5, **characterized in that** it contains a mixture of mild anionic and mild amphoteric and/or zwitterionic surfactants as the surfactant combination.

7. The care emulsion according to claim 6, **characterized in that** the surfactants are selected from one or more surfactants of the group (iii) of the acyl amino acids, alpha-olefin sulfonates, ethoxylated alkyl sulfuric acid esters, sulfosuccinic acid esters, ether carboxylic acids and/or the salts of these compounds, and from one or more surfactants of the group (iv) of the alkyl betaines, alkylamido alkyl betaines, aminopropionates, amino glycinates, sulfobetaines and/or imidazolinium betaines and/or the salts of these compounds.

8. The care emulsion according to one of claims 1 to 7, **characterized in that** a combination of dicaprylyl ether, ethylhexyl palmitate and C₁₂-C₁₅ alkyl benzoate is contained as the oil or fat component.

9. The care emulsion according to one of claims 1 to 8, **characterized in that**, in order to increase the emulsion and foam stability, it contains a combination of at least two polymers, of which one is of natural origin and the other is of synthetic origin.

10. The care emulsion according to claim 9, **characterized in that** the polymers of synthetic origin are selected from polyacrylic acids and/or polyacryloyldimethyl taurates, and the polymers of natural origin are selected from xanthan gum derivatives, guar gum derivatives, alginate derivatives and/or carrageenan derivatives.

11. The care emulsion according to one of claims 1 to 10, **characterized in that** it contains a combination of different emulsion systems.

12. The care emulsion according to one of claims 1 to 11, **characterized in that** it contains at least one further cosmetic auxiliary or active substance that is selected from the group of the sunprotection agents, humectants, antioxidants, hyaluronic acid and the physiologically acceptable salts and derivatives thereof, active substances for stimulating collagen synthesis, active substances for increasing or improving the interaction between the extracellular matrix and the fibroblasts, and active substances for promoting elastin production.

13. The care emulsion according to one of claims 1 to 12, **characterized in that** it has a pH in the range between 3.5 and 7.5, preferably 4 and 7 and in particular 4.5 and 6.5.

14. The use of a mixture of an emulsifier combination (A), containing two W/O emulsifiers of the same HLB value that are selected from
(i) at least one poly(12-hydroxystearic acid)polyglycerol ester and
(ii) at least one silicone emulsifier,
a surfactant combination (B) containing
(iii) at least one anionic surfactant and
(iv) at least one amphoteric and/or zwitterionic surfactant, and
1-25 wt.% of at least one oil or fat component, based on the total weight of the care emulsion, for producing cosmetic care emulsions that can be foamed using a manual pump dispenser and that have a light texture and a better distribution capability.

15. The use of a care emulsion according to one of claims 1 to 13 for improving the feeling of the skin.

## Revendications

1. Émulsion de soin moussante quand on utilise un distributeur manuel à pompe, contenant :
(A) une combinaison d'émulsifiants i) + ii) constituée d'au moins deux émulsifiants huile dans l'eau de mêmes valeurs HLB, choisis parmi
(i) au moins un ester polyglycérique d'acide poly(12-hydroxystéarique) et
(ii) au moins un émulsifiant silicone, et
(B) une combinaison de tensioactifs iii) + iv) constituée de :
(iii) au moins un tensioactif anionique, et
(iv) au moins un tensioactif amphotère et/ou zwitterionique, et
(C) 1 à 25 % en poids rapportés au poids total de l'émulsion de soin d'au moins un composant huile ou graisse.

2. Émulsion de soin selon la revendication 1, **caractérisée en ce que** les valeurs HLB des émulsifiants se situent dans la gamme entre 2 et 8, préférentiellement entre 3 et 7 et en particulier dans la gamme entre 4 et 6.

3. Émulsion de soin selon la revendication 1 ou la revendication 2, **caractérisée en ce qu'**elle contient les émulsifiants i) et ii) dans un rapport pondéral de 5:1 à 1:4, de préférence de 3:1 à 1:2 et en particulier de 2:1 à 1:1.

4. Émulsion de soin selon l'une des revendications 1 à 3, **caractérisée en ce qu'**elle contient comme émulsifiant silicone un organopolysiloxane-polyoxyde d'alkylène.

5. Émulsion de soin selon la revendication 4, **caractérisée en ce que** l'émulsifiant silicone contient un groupe alkyle en C8-18 ainsi qu'un ou plusieurs groupes éthoxy et/ou propoxy.

6. Émulsion de soin selon l'une des revendications 1 à 5, **caractérisée en ce qu'**elle contient comme combinaison de tensioactifs un mélange de tensioactifs anioniques doux et amphotères et/ou zwitterioniques doux.

7. Émulsion de soin selon la revendication 4, **caractérisée en ce que** les tensioactifs sont choisis parmi un ou plusieurs tensioactifs du groupe (iii) des acylaminoacides, α-sulfonates d'oléfines, sulfates d'alkyle éthoxylés, esters de l'acide sulfosuccinique, éther-acides carboxyliques et/ou des sels de ces composés, ainsi que parmi un ou plusieurs tensioactifs du groupe (iv) des alkylbétaïnes, alkylamidoalkylbétaïnes, aminopropionates, aminoglycinates, sulfobétaïnes et/ou imidazolinium bétaïnes et/ou des sels de ces composés.

8. Émulsion de soin selon l'une des revendications 1 à 7, **caractérisée en ce qu'**elle contient comme composés huiles ou graisses une combinaison de dicapryléther, palmitate d'éthylhexyle et de benzoate d'alkyles en C₁₂₋₁₅.

9. Émulsion de soin selon l'une des revendications 1 à 8, **caractérisée en ce qu'**elle contient, pour augmenter la stabilité de l'émulsion et de la mousse, une combinaison d'au moins deux polymères, dont un est d'origine naturelle et l'autre est d'origine synthétique.

10. Émulsion de soin selon la revendication 9, **caractérisée en ce que** les polymères d'origine synthétique sont choisis parmi des polyacrylates et/ou des polyacryloyldiméthyltaurates ; et les polymères d'origine naturelle sont choisis parmi des dérivés de gomme xanthane, gomme de guar, alginate et/ou carraghénanes.

11. Émulsion de soin selon l'une des revendications 1 à 10, **caractérisée en ce qu'**elle comprend une combinaison de différents systèmes d'émulsions.

12. Émulsion de soin selon l'une des revendications 1 à 11, **caractérisée en ce qu'**elle contient au moins un autre adjuvant ou agent actif cosmétique choisi dans le groupe des agents de protection solaire, des agents d'humidification, des antioxydants, de l'acide hyaluronique, ainsi que de leurs sels et dérivés physiologiquement acceptables, d'agents de stimulation de la synthèse du collagène, d'agents d'augmentation ou d'amélioration de l'interaction entre la matrice extracellulaire et les fibroblastes, ainsi que d'agents favorisant la production d'élastine.

13. Émulsion de soin selon l'une des revendications 1 à 12, **caractérisée en ce qu'**elle présente un pH dans la gamme entre 3,5 et 7,5, de préférence entre 4 et 7, et en particulier entre 4,5 et 6,5.

14. Utilisation d'un mélange d'une combinaison d'émulsifiants A) contenant deux émulsifiants eau dans l'huile de mêmes valeurs HLB, choisis parmi :
(i) au moins un poly(12-hydroxystéarate) de polyglycérine et
(ii) au moins un émulsifiant silicone,
d'une combinaison de tensioactifs B) contenant :
(iii) au moins un tensioactif anionique et
(iv) au moins un tensioactif amphotère et/ou zwitterionique, et
1 à 25 % en poids rapportés au poids total de l'émulsion de soin d'au moins un composant huile ou graisse, pour la fabrication d'émulsions de soin cosmétiques moussantes quand on utilise un distributeur manuel à pompe et qui présentent une texture légère ainsi qu'une meilleure capacité d'étalement.

15. Utilisation d'une émulsion de soin selon l'une des revendications 1 à 13 pour améliorer le toucher de la peau.
